(19) Europäisches Patentamt
European Patent Office
Office européen des brevets

(11) Veröffentlichungsnummer: **0 007 438**
**B1**

(12) **EUROPÄISCHE PATENTSCHRIFT**

(45) Veröffentlichungstag der Patentschrift: **01.09.82**

(51) Int. Cl.³: **C 07 D 233/50**, A 61 K 31/415

(21) Anmeldenummer: **79102052.2**

(22) Anmeldetag: **21.06.79**

(54) **Neue substituierte 2-Phenylamino-imidazoline-(2), deren Säureadditionssalze, diese enthaltende Arzneimittel und Verfahren zur Herstellung derselben.**

(30) Priorität: **19.07.78 DE 2831671**

(43) Veröffentlichungstag der Anmeldung:
**06.02.80 Patentblatt 80/3**

(45) Bekanntmachung des Hinweises auf die Patenterteilung:
**01.09.82 Patentblatt 82/35**

(84) Benannte Vertragsstaaten:
**AT BE CH DE FR GB IT LU NL SE**

(56) Entgegenhaltungen:
**FR - A - 2 122 580**

Die Akte enthält technische Angaben die nach dem Eingang der Anmeldung eingereicht wurden und die nicht in dieser Patentschrift enthalten sind.

(73) Patentinhaber: **C.H. BOEHRINGER SOHN**
**Postfach 200**
**D-6507 Ingelheim am Rhein (DE)**

(72) Erfinder: **Stähle, Helmut, Dr.**
**Rotweinstrasse 23**
**D-6507 Ingelheim (DE)**
Erfinder: **Köppe, Herbert, Dr.**
**Neuweg 72**
**D-6507 Ingelheim (DE)**
Erfinder: **Kummer, Werner, Dr.**
**Georg-Scheuing-Strasse 15**
**D-6507 Ingelheim (DE)**
Erfinder: **Kobinger, Walter, Prof. Dr.**
**Belghofergasse 27**
**A-1120 Wien (AT)**
Erfinder: **Lillie, Christian, Dr. Mag.**
**Hansi-Niese-Weg 12**
**A-1130 Wien (AT)**
Erfinder: **Pichler, Ludwig, Dr.**
**Gusshausstrasse 24**
**A-1040 Wien (AT)**

**EP 0 007 438 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Neue substituierte 2-Phenylamino-imidazoline-(2), deren Säureadditionssalze, diese enthaltende Arzneimittel und Verfahren zur Herstellung derselben

Aus der DE—OS 1 958 201 und der FR—PS 2 122 580 sind N-Allyl-2-arylamino-imidazoline bekannt, die blutdrucksenkend und analgetisch wirken. Es wurde nun gefunden, daß neue Verbindungen ähnlicher Konstitution überraschenderweise eine gegenüber den bekannten Derivaten des Standes der Technik überlegene bradycarde Wirksamkeit aufweisen.

Die Erfindung betrifft neue substituierte 2-Phenylaminoimidazoline-(2) der allgemeinen Formel

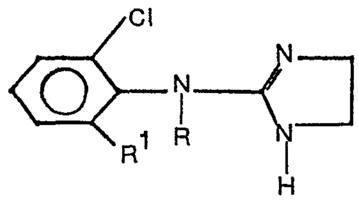

I

sowie deren physiologisch verträgliche Säureadditionssalze mit wertvollen therapeutischen Eigenschaften.

In der Formel I bedeuten $R^1$ ein Chloratom oder eine Methylgruppe und R den Rest $-(CH_2)_2-C(CH_3)=CH_2$, $-(CH_2)_2-CH=CH_2$, $-O-CH_2-CH=CH_2$, $-O-CH_2-C(CH_3)=CH_2$ oder $-O-CH_2-CH=CH-CH_3$.

Die Herstellung der Verbindungen der Formel I erfolgt durch:

a) Umsetzung eines 2-Phenylimino-imidazolidins der allgemeinen Formel

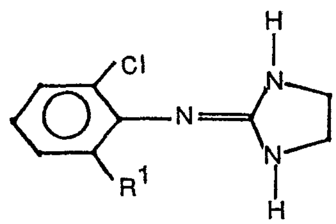

II

in der $R^1$ die oben genannten Bedeutungen besitzt, mit einem Halogenid der allgemeinen Formel

Hal—R III

worin Hal ein Chlor-, Brom oder Jodatom bedeutet und R die obige Bedeutung hat; oder

b) Umsetzung einer Verbindung der allgemeinen Formel

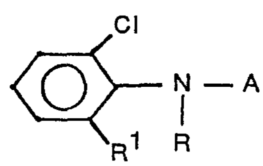

IV

in der $R^1$ und R wie oben angegeben definiert sind und A eine Cyanogruppe oder den Rest

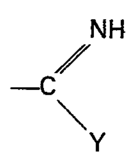

bedeutet, wobei Y eine Alkoxy- oder Alkylthiogruppe darstellt, mit Äthylendiamin oder dessen Säureadditionssalzen.

c) Zwecks Herstellung von 2-[N-(subst.-Phenyl)-N-(subst.-methoxyamino]-imidazolinen-(2) der allgemeinen Formel

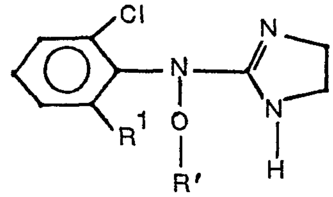

V

worin $R^1$ die obige Bedeutung hat und R′ die Teilformel $-CH_2-CH=CH_2$, $-CH_2-C(CH_3)=CH_2$ oder $-CH_2-CH=CH-CH_3$ besitzt, setzt man ein 2-[N-(subst.-Phenyl)-N-hydroxy-amino]-imidazolin-(2) der allgemeinen Formel

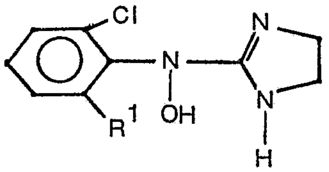

VI

worin $R^1$ wie oben angegeben definiert ist, mit einem Halogenid der allgemeinen Formel

$$Hal-R'$$

VII

worin Hal und $R^1$ die oben genannten Bedeutungen haben, um.

Bei der Alkylierung der 2-Arylamino-imidazolidine der Formel II nach Verfahren a) erfolgt die Substitution ausschließlich am Brückenstickstoffatom. Bei der Umsetzung nach den Verfahren b) und c) ist die Konstitution der Endverbindungen durch die Synthese festgelegt. Die Position der Substituenten läßt sich außer durch die Synthese auch durch die NMR-Spektroskopie festlegen. (Vgl. H. Stähle und K.—H. Pook, Liegibs Ann. Chem. 751, 159 ff (1971).

Die Umsetzung nach Verfahren a) und c) erfolgt zweckmäßigerweise durch Erhitzen der Reaktionspartner — vorzugsweise in Gegenwart eines polaren oder unpolaren organischen Lösungsmittels — auf Temperaturen von etwa 50 bis 150°C. Die speziellen Reaktionsbedingungen hängen in starkem Maße von der Reaktivität der Umsetzungsteilnehmer ab. Es empfiehlt sich, bei der Alkylierung das Halogenid im Überschuß anzuwenden und die Umsetzung in Gegenwart eines säurebindenden Mittels durchzuführen.

Bei dem Verfahren nach b) ist es erforderlich, bei erhöhter Temperatur, zwischen 60°C und 180°C zu arbeiten. Lösungsmittel sind nicht erforderlich. Es ist zweckmäßig, das als Reaktionspartner vorzugsweise Äthylendiamin bzw. dessen Säureadditionssalz, im Überschuß anzuwenden.

Ausgangsverbindungen der Formel II sing z.B. in den belgischen Patenten Nr. 623 305, 687 657 und 705 944 beschrieben.

Ausgangsverbindungen der Formeln III und VII lassen sich durch Halogenierung der zugrundeliegenden primären Alkohole darstellen.

Verbindungen der Formel IV erhält man ausgehend von Anilinen, durch Umsetzung mit Verbindungen der Formel III und nachfolgende Reaktion der dabei entstehenden sekundären Amine mit Cyanaten oder Thiocyanaten, wobei Harnstoffe, bzw. Thioharnstoffe gebildet werden. Harnstoffe und Thioharnstoffe können dann weiter mit Alkylierungsmitteln in entsprechende Isouroniumsalze bzw. Isothiouroniumsalze überführt werden. Aus diesen Säureadditionsverbindungen lassen sich mit Basen die entsprechenden Isoharnstoffe bzw. Isothioharnstoffe gewinnen. Durch Wasserabspaltung aus Harnstoffen bzw. $H_2S$-Abspaltung aus Thioharnstoffen mittels Blei- oder Quecksilbersalzen gelangt man zu Cyanamiden, an die Ammoniak unter Bildung von Guanidinen angelagert werden kann.

Die Ausgangsverbindungen der Formel VI erhält man durch Oxydation von Verbindungen der allgemeinen Formel II mit Persäuren entsprechend DE—OS 24 57 979.

Die erfindungsgemäßen 2-Phenylamino-imidazoline-(2) der allgemeinen Formel I können auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen.

Die neuen Verbindungen der allgemeinen Formel I und deren Säureadditionssalze wirken sehr stark bradycard und sind daher zur Therapie von Coronarerkrankungen und somit von Tachycardien beeignet. Die Beeinflussung der Herzfrequenz wurde an Kaninchen und an Spinalratten sowie an intakten, narkotisierten Ratten untersucht. Die Dosierung liegt bei 0,1 bis 80 mg, vorzugsweise 1 bis 30 mg.

Die Verbindungen der Formel I bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele erläutern die Erfindung, ohne sie zu beschränken.

Beispiel 1

2-[N-(2,6-Dichlorphenyl)-N-(2-methyl-1-buten-4-yl)-amino]-2-imidazolin

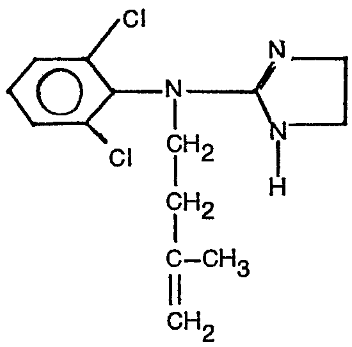

6,9 g (0.03 Mol) Clondin werden zusammen mit 5,6 g 4-Brom-2-methyl-buten-(1) und 5 ml Triäthylamin in 25 ml Toluol 24 Stunden lang im Rohr auf 120°C erhitzt. Nach dem Erkalten wird das Lösungsmittel abdekantiert und der verbleibende Rückstand in 1 N Salzsäure gelöst. Nach mehrmaligem Ausäthern (Ätherextrakte werden verworfen) wird bei aufsteigenden pH-Werten (Alkalisieren mit 2N Natronlauge) fraktioniert mit Äther extrahiert. Die dünnschichtchromatografisch einheitlichen Ätherextrakte werden vereinigt, über $MgSO_4$ getrocknet und der Äther im Vakuum abgezogen. Ausbeute 1,8 g (19,6% der Theorie) Schmelzpunkt: 117—119°C.

Analog dem vorstehenden Beispiel wurden die folgenden Verbindungen hergestellt. Die Schmelzpunkte beziehen sich auf die Basen der Formel I.

| Beispiel Nr. | $R^1$ | R | Fp.[°C] | Ausbeute % der Theorie |
|---|---|---|---|---|
| 2 | Cl | $—(CH_2)_2—CH{=}CH_2$ | 133—135 | 39,6 |
| 3 | Cl | $—O—CH_2—CH{=}CH_2$ | 153—154 | 61,6 |
| 4 | Cl | $—O—CH_2—C(CH_3){=}CH_2$ | 112—113 | 18,4 |
| 5 | Cl | $—O—CH_2—CH{=}CH—CH_3$ | 135—138 | 47,6 |
| 6 | $CH_3$ | $—(CH_2)_2—C(CH_3){=}CH_2$ | 109—111 | 19,3 |

*Formulierungsbeispiele*

Beispiel A: Dragées

| | |
|---|---|
| Wirkstoff gemäß Erfindung | 5 mg |
| Milchzucker | 65 mg |
| Maisstärke | 130 mg |
| sek. Calciumphosphat | 40 mg |
| lösliche Stärke | 3 mg |
| Magnesiumstearat | 3 mg |
| kolloidale Kieselsäure | 4 mg |
| insgesamt | 250 mg |

Herstellung:

Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht verpreßt, die dann in üblicher Weise mit Hilfe von Zucker, Talkum und Gummi arabicum dragiert werden.

Beispiel B: Ampullen

| | | |
|---|---|---|
| Wirkstoff gemäß Erfindung | | 1,0 mg |
| Natriumchlorid | | 18,0 mg |
| dest. Wasser | ad | 2,0 ml |

Herstellung:

Wirkstoff und Natriumchlorid werden in Wasser gelöst und unter Stickstoff in Glasampullen abgefüllt.

Beispiel C: Tropfen

| | | |
|---|---|---|
| Wirkstoff gemäß Erfindung | | 0,02 g |
| p-Hydroxybenzoesäuremethylester | | 0,07 g |
| p-Hydroxybenzoesäurepropylester | | 0,03 g |
| entmineralisiertes Wasser | ad | 100 ml |

**Patentansprüche für die Vertragsstaaten BE, CH, DE, FR, GB, IT, LU, NL, SE**

1. Substituierte 2-Phenylamino-imidazoline-(2) der allgemeinen Formel

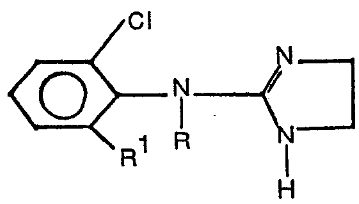

I

worin $R^1$ ein Chloratom oder eine Methylgruppe und R den Rest $-(CH_2)_2-C(CH_3)=CH_2$, $-(CH_2)_2-CH=CH_2$, $-O-CH_2-CH=CH_2$, $-O-CH_2-C(CH_3)=CH_2$ oder $-O-CH_2-CH=CH-CH_3$ bedeutet sowie deren Säureadditionssalze.

2. Verfahren zur Herstellung von substituierten 2-Phenylamino-imidazolinen-(2) der allgemeinen Formel I nach Anspruch 1 und deren Säureadditionssalzen, dadurch gekennzeichnet, daß man

a) ein 2-Phenylimino-imidazolidin der allgemeinen Formel

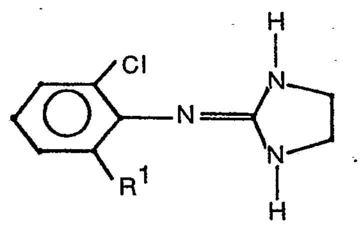

II

in der $R^1$ die oben genannten Bedeutungen Besitzt, mit einem Halogenid der allgemeinen Formel

$$Hal-R \qquad III$$

worin Hal ein Chlor-, Brom- oder Jodatom bedeutet und R die genannte Bedeutung hat, umsetzt oder

b) eine Verbindung der allgemeinen Formel

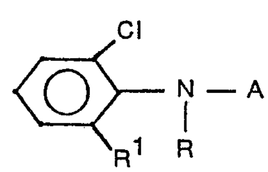

IV

in der $R^1$ und R wir oben angegeben definiert sind und A eine Cyanogruppe oder den Rest

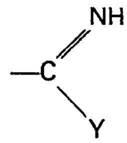

bedeutet, wobei Y eine Alkoxy- oder Alkylthiogruppe mit bis zu 4 Kohlenstoffatomen oder eine Sulfhydryl- oder Aminogruppe darstellt, mit Äthylendiamin oder dessen Säureadditionssalzen umsetzt oder

c) zwecks Herstellung von 2-[N-(subst.-Phenyl)-N-(subst.-methoxy-amino]-imidazolinen-(2) der allgemeinen Formel

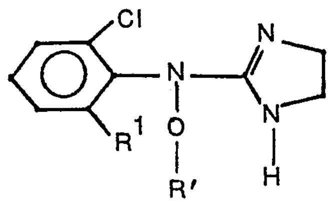

V

worin $R^1$ die obige Bedeutung hat und R' die Teilformel $—CH_2—CH=CH_2$, $—CH_2—C(CH_3)=CH_2$ oder $—CH_2—CH=CH—CH_3$ besitzt, ein 2-[N-(subst.-Phenyl)-N-hydroxy-amino]-imidazolin-(2) der allgemeinen Formel

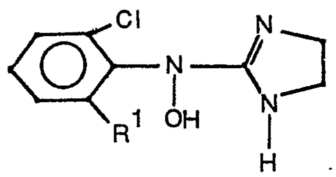

VI

worin $R^1$ wie oben angegeben definiert ist, mit einem Halogenid der allgemeinen Formel

Hal—R' VII

worin Hal und R' die oben genannten Bedeutungen haben, umsetzt und daß man gegebenenfalls das nach einem der Verfahren erhaltene Endprodukt in ein Säureadditionssalz überführt.

3. Verfahren nach Anspruch 2, dadurch gekennzeichnet, daß man die Umsetzung bei Temperaturen von 50 bis 150 bzw. 60 bis 180°C durchführt, wobei letztgenannter Bereich für die Verfahrensvariante b), der erstgenannte Bereich für die beiden Varianten a) und c) gilt.

4. Verfahren nach Anspruch 2 a) und c) und/oder 3, dadurch gekennzeichnet, daß man die Umsetzung in Gegenwart eines polaren oder unpolaren organischen Lösungsmittels durch führt.

5. Verfahren nach Anspruch 2 a) und c) bis 4, dadurch gekennzeichnet, daß man die Umsetzung in Gegenwart eines säurebindenden Mittels durchführt.

6. Pharmazeutische Zubereitungen, dadurch gekennzeichnet, daß sie als Wirkstoff eine oder mehrere Verbindungen der allgemeinen Formel I nach Anspruch 1 oder deren Säureadditionssalze enthalten.

7. Verfahren zur Herstellung von Arzneimitteln nach Anspruch 6, dadurch gekennzeichnet, daß man eine oder mehrere Verbindungen der allgemeinen Formel I oder deren Säureadditionssalze mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

8. Verbindungen der allgemeinen Formel I nach Anspruch 1 oder deren physiologisch verträgliche Säureadditionssalze zur Verwendung bei der Behandlung von Tachycardien.

9. Verwendung von Verbindungen der allgemeinen Formel I nach Anspruch 1 oder von deren physiologisch verträglichen Säureadditionssalzen zur Herstellung von Arzneimitteln für die Behandlung von Tachycardien.

**Patentansprüche für den Vertragsstaat AT**

1. Verfahren zur Herstellung von substituierten 2-Phenylamino-imidazoline-(2) der allgemeinen Formel

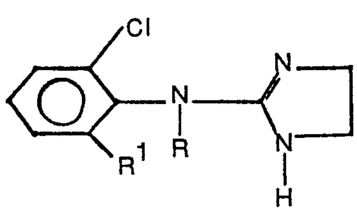

I

worin $R^1$ ein Chloratom oder eine Methylgruppe und R den Rest $-(CH_2)_2-C(CH_3)=CH_2$, $-(CH_2)_2-CH=CH_2$, $-O-CH_2-CH=CH_2$, $-O-CH_2-C(CH_3)=CH_2$ oder $-O-CH_2-CH=CH-CH_3$ bedeutet sowie von deren Säureadditionssalzen, dadurch gekennzeichnet, daß man

a) ein 2-Phenylimino-imidazolidin der allgemeinen Formel

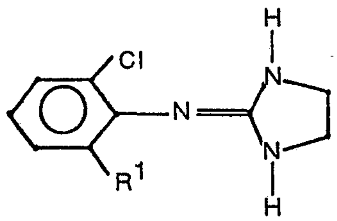

II

in der $R^1$ die oben genannten Bedeutungen Besitzt, mit einem Halogenid der allgemeinen Formel

Hal—R III

worin Hal ein Chlor-, Brom- oder Jodatom bedeutet und R die genannte Bedeutung hat, umsetzt oder

b) eine Verbindung der allgemeinen Formel

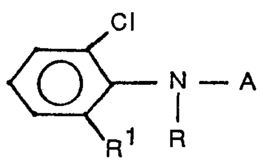

IV

in der $R^1$ und R wie oben angegeben definiert sind und A eine Cyanogruppe oder den Rest

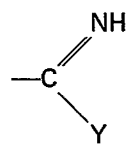

bedeutet, wobei Y eine Alkoxy- oder Alkylthiogruppe mit bis zu 4 Kohlenstoffatomen oder eine Sulfhydryl- oder Aminogruppe darstellt, mit Äthylendiamin oder dessen Säureadditionssalzen umsetzt oder

c) zwecks Herstellung von 2-[N-(subst.-Phenyl)-N-(subst.-methoxy-amino]-imidazolinen-(2) der allgemeinen Formel

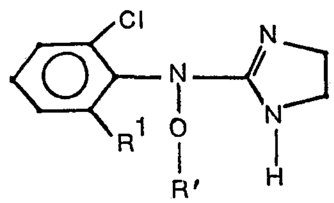

V

worin $R^1$ die obige Bedeutung hat und R′ die Teilformel $-CH_2-CH=CH_2$, $-CH_2-C(CH_3)=CH_2$ oder $-CH_2-CH=CH-CH_3$ besitzt, ein 2-[N-(subst.-Phenyl)-N-hydroxy-amino]-imidazolin-(2) der allgemeinen Formel

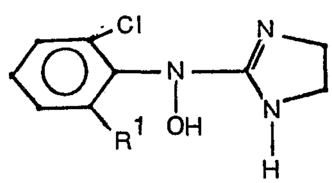

VI

worin $R^1$ wie oben angegeben definiert ist, mit einem Halogenid der allgemeinen Formel

Hal—R′ VII

worin Hal und R′ die oben genannten Bedeutungen haben, umsetzt und daß man gegebenenfalls das nach einem der Verfahren erhaltene Endprodukte in ein Säureadditionssalz überführt.

2. Verfahren nach Anspruch 1, dadurch gekennzeichnet, daß man die Umsetzung bei Temperaturen von 50 bis 150°C bzw. 60 bis 180°C durchführt, wobei letztgenannter Bereich für die Verfahrensvariante b), der erstgenannte Bereich für die beiden Varianten a) und c) gilt.

3. Verfahren nach Anspruch 1 a) und c), dadurch gekennzeichnet, daß man die Umsetzung in Gegenwart eines polaren oder unpolaren organischen Lösungsmittels durchführt.

4. Verfahren nach Anspruch 1 a) und c) bis 3, dadurch gekennzeichnet, daß man die Umsetzung in Gegenwart eines säurebindenden Mittels durchführt.

5. Verfahren zur Herstellung von Arzneimitteln, dadurch gekennzeichnet, daß man eine oder mehrere Verbindungen der allgemeinen Formel I nach Anspruch 1 oder deren Säureadditionssalze mit üblichen galenischen Hilfs-, Träger-, Spreng- oder Schmiermitteln bzw. Substanzen zur Erzielung einer Depotwirkung zu Tabletten, Kapseln, Zäpfchen, Lösungen oder Pulver formuliert.

6. Verwendung von Verbindungen der allgemeinen Formel I nach Anspruch 1 oder von deren physiologisch verträglichen Säureadditionssalzen zur Herstellung von Arzneimitteln für die Bekämpfung von Tachycardien.

**Revendications pour les Etats contractants désignés BE, CH, DE, FR, GB, IT, Lu, NL, SE**

1. 2-phénylamino-imidazolines-(2) substituées de formule générale

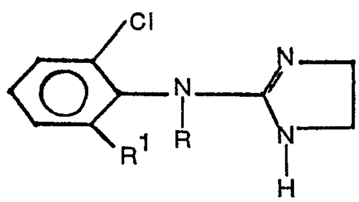

I

dans laquelle $R^1$ represente un atome de chlore ou un groupe méthyle et R représente le radical $-(CH_2)_2-C(CH_3)=CH_2$, $-(CH_2)_2-CH=CH_2$, $-O-CH_2-CH=CH_2$, $-O-CH_2-C(CH_3)=CH_2$ ou $-O-CH_2-CH=CH-CH_3$ ainsi que leurs sels d'addition avec des acides.

2. Procédé pour la préparation des 2-phénylamino-imidazolines-(2) substituées de formule générale I selon la revendication 1 et de leurs sels d'addition avec des acides, caractérisé en ce que

a) on fait réagir une 2-phénylimino-imidazolidine de formule générale

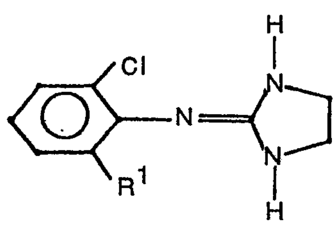

II

dans laquelle $R^1$ possède les significations mentionnées plus haut, avec un halogénure de formule générale

$$Hal-R \qquad III$$

dans laquelle Hal représente un atome de chlore, de brome ou d'iode et R a la signification mentionnée, ou

b) on fait réagir un composé de formule générale

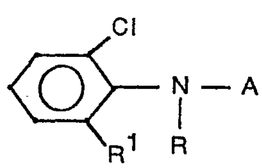

IV

dans laquelle $R^1$ et R sont définis comme indiqué plus haut et A représente un groupe cyano ou le radical

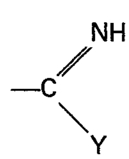

Y représentant un groupe alcoxy ou alcoylthio avec jusqu'à 4 atomes de carbone ou un gorupe sulfhydryle ou amino, avec l'éthylènediamine ou ses sels d'addition avec des acides ou

c) pour la préparation des 2-[N-(phényl-subst.)-N-(méthoxy-subst.amino]-imidazolines-(2) de formule générale

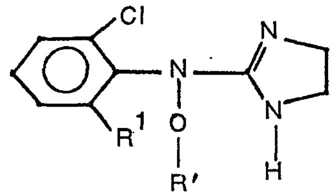

V

dans laquelle $R^1$ a la signification ci-dessus et R' possède la formule partielle $—CH_2—CH=CH_2$, $—CH_2—C(CH_3)=CH_2$ ou $—CH_2—CH=CH—CH_3$, on fait réagir une 2-[N-(phényl-subst.)-N-hydroxyamino]-imidazoline-(2) de formule générale

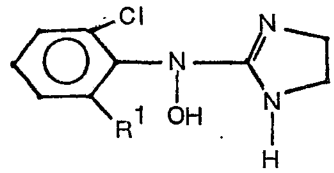

VI

dans laquelle $R^1$ est défini comme indiqué plus haut, avec un halogénure de formule générale

Hal—R' VII

dans laquelle Hal et R' ont les significations mentionnées plus haut, et en ce qu'on transforme éventuellement le produit final obtenu selon l'un des procédés en un sel d'addition avec un acide.

3. Procédé selon la revendication 2, caractérise en ce qu'on effectue la réaction à des températures de 50 à 150°C ou respectivement de 60 à 180°C, le domaine mentionné en dernier convenant pour la variante de procédé b), le domaine mentionné en premier pour les deux variantes a) et c).

4. Procédé selon la revendication 2 a) et c) et/ou 3, caractérisé en ce qu'on effectue la réaction dans un solvant organique polaire ou non polaire.

5. Procédé selon la revendication 2 a) et c) à 4, caractérisé en ce qu'on effectue la réaction en présence d'un agent fixant les acides.

6. Préparations pharmaceutiques, caractérisées en ce qu'elles contiennent en tant que substance active un ou plusieurs composés de formule générale I selon la revendication 1 ou leurs sels d'addition avec des acides.

7. Procédé pour la préparation de médicaments selon la revendication 6, caractérisé en ce qu'on formule un ou plusieurs composés de formule générale I ou leurs sels d'addition avec des acides avec des adjuvants, excipients, désagrégeants ou lubrifiants ou respectivement substances pour obtenir un effet retard galéniques habituels, en comprimés, capsules, suppositoires, solutions ou poudres.

8. Composés de formule générale I ou leurs sels d'addition avec des acides physiologiquement supportables pour l'utilisation dans le traitement des tachycardies.

9. Utilisation des composés de formule générale I selon la revendication 1 ou de leurs sels d'addition avec des acides physiologiquement supportables pour la préparation de médicaments pour le traitement des tachycardies.

**Revendications pour l'Etat contractant désigné AT**

1. Procédé pour la préparation de 2-phénylamino-imidazolines-(2) substituées de formule générale

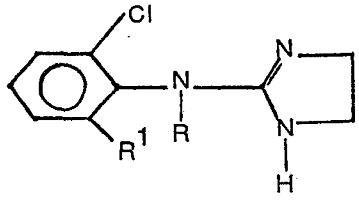

I

dans laquelle $R^1$ représente un atome de chlore ou un groupe méthyle et R représente le radical

$—(CH_2)_2—C(CH_3)=CH_2$, $—(CH_2)_2—CH=CH_2$, $—O—CH_2—CH=CH_2$, $—O—CH_2—C(CH_3)=CH_2$ ou $—O—CH_2—CH=CH—CH_3$ ainsi que de leurs sels d'addition avec des acides, caractérisé en ce que

a) on fait réagir une 2-phénylimino-imidazolidine de formule générale

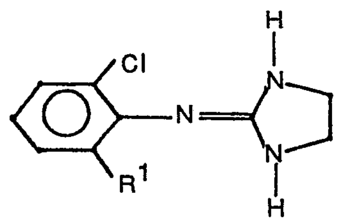

II

dans $R^1$ possède les significations mentionnées plus haut, avec un halogénure de formule générale

Hal—R III

dans laquelle Hal représente un atome de chlore, de brome ou d'iode et R a la signification mentionnée, ou

b) on fait réagir un composé de formule générale

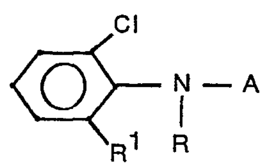

IV

dans laquelle $R^1$ et R sont définis comme indiqué plus haut et A représente un groupe cyano ou le radical

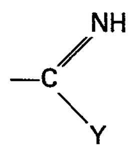

Y représentant un groupe alcoxy ou alcoylthio avec jusqu'à 4 atomes de carbone ou un groupe sulfhydryle ou amino, avec l'éthylènediamine ou ses sels d'addition avec des acides ou

c) pour la préparation des 2-[N-(phényl-subst.)-N-(méthoxy-subst.amino]-imidazolines-(2) de formule générale

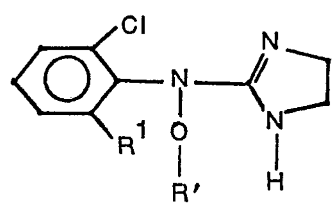

V

dans laquelle $R^1$ a la signification ci-dessus et R′ possède la formule partielle $—CH_2—CH=CH_2$, $—CH_2—C(CH_3)=CH_2$ ou $—CH_2—CH=CH—CH_3$, on fait réagir une 2-[N-(phényl-subst.)-N-hydroxy-amino]-imidazoline-(2) de formule générale

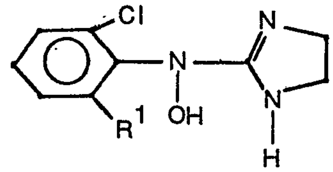

VI

dans laquelle $R^1$ est défini comme indiqué plus haut, avec un halogénure de formule générale

Hal—R′ VII

dans laquelle Hal et R′ ont les significations mentionnées plus haut, et en ce qu'on transforme éventuellement le produit final obtenu selon l'un des procédés en un sel d'addition avec un acide.

2. Procédé selon la revendication 1, caractérise en ce qu'on effectue la réaction à des températures de 50 à 150°C ou respectivement de 60 à 180°C, le domaine mentionné en dernier étant valable pour la variante de procédé b), le domaine mentionné en premier pour les deux variantes a) et c).

3. Procédé selon la revendication 1 a) et c), caractérisé en ce qu'on effectue la réaction en présence d'un solvant organique polaire ou non polaire.

4. Procédé selon la revendication 1 a) et c) à 3, caractérisé en ce qu'on effectue la réaction en présence d'un agent fixant les acides.

5. Procédé pour la préparation de médicaments, caractérisé en ce qu'on formule un ou plusieurs composés de formule générale I selon la revendication 1 ou leurs sels d'addition avec des acides avec des adjuvants, excipients, désagrégeants ou lubrifiants ou respectivement substances pour obtenir un effet retard galéniques habituels, en comprimés, capsules, suppositoires, solutions ou poudres.

6. Utilisation des composés de formule générale I selon la revendication 1 ou de leurs sels d'addition avec des acides et physiologiquement supportables pour la préparation de médicaments pour le traitement des tachycardies.

**Claims for the designated contracting states BE, CH, DE, FR, GB, IT, LU, NL, SE**

1. Substituted 2-phenylamino-imidazolines-(2) of general formula

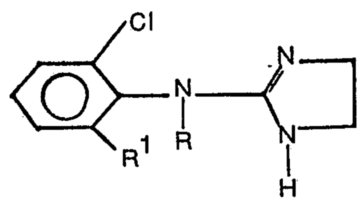

I

wherein $R^1$ represents a chlorine atom or a methyl group and R represents the group $-(CH_2)_2-C(CH_3)=CH_2$, $-(CH_2)_2-CH=CH_2$, $-O-CH_2-CH=CH_2$, $-O-CH_2-C(CH_3)=CH_2$ or $-O-CH_2-CH=CH-CH_3$ and the acid addition salts thereof.

2. Process for the preparation of substituted 2-phenylamino-imidazolines-(2) of general formula 1 as claimed in claim 1 and the acid addition salts thereof, characterised in that

a) a 2-phenylimino-imidazolidine of general formula

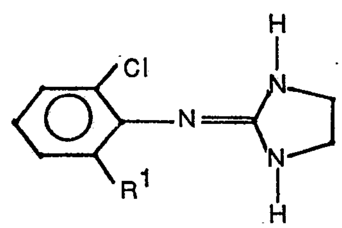

II

wherein $R^1$ is as hereinbefore defined, is reacted with a halide of general formula

$$Hal-R \qquad III$$

wherein Hal represents a chlorine, bromine or iodine atom and R is as hereinbefore defined, or

b) a compound of general formula

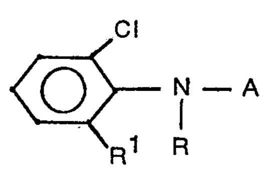

IV

wherein $R^1$ and R are as hereinbefore defined and A represents a cyano group or the group

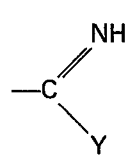

wherein Y represents an alkoxy or alkylthio group with up to 4 carbon atoms or a mercapto or amino group, is reacted with ethylenediamine or the acid addition salts thereof, or

c) to prepare 2-[N-(substituted phenyl)-N-(substituted methoxy-amino]-imidazolines-(2) of general formula

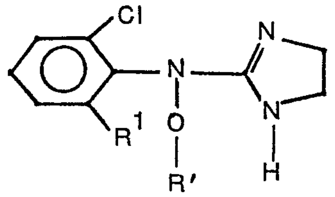

V

wherein $R^1$ is as hereinbefore defined and R′ has the partial formula $—CH_2—CH=CH_2$, $—CH_2—C(CH_3)=CH_2$ or $—CH_2—CH=CH—CH_3$, a 2-[N-(substituted phenyl)-N-hydroxyamino]-imidazoline-(2) of general formula

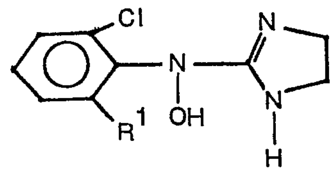

VI

wherein $R^1$ is as hereinbefore defined, is reacted with a halide of general formula

Hal—R′ VII

wherein Hal and R′ are as hereinbefore defined, and if desired the end product obtained according to one of these processes is converted into an acid addition salt.

3. Process as claimed in claim 2, characterised in that the reaction is effected at temperatures of 50 to 150 or 60 to 180°C, the latter range applying to variant b) of the process, whilst the former range applies to the two variants a) and c).

4. Process as claimed in claim 2 a) and c) and/or 3, characterised in that the reaction is carried out in the presence of a polar or apolar organic solvent.

5. Process as claimed in claim 2 a) and c) to 4, characterised in that the reaction is carried out in the presence of an acid-binding agent.

6. Pharmaceutical preparations, characterised in that they contain, as active ingredient, one or more compounds of general formula I according to claim 1 or the acid addition salts thereof.

7. Process for the preparation of pharmaceutical compositions as claimed in claim 6, characterised in that one or more compounds of general formula I or the acid addition salts thereof are combined with conventional galenic excipients, carriers, disintegrants or lubricants or substances for obtaining delayed release, to make tablets, capsules, suppositories, solutions or powders.

8. Compounds of general formula I as claimed in claim 1 or the physiologically acceptable acid addition salts thereof for use in the treatment of tachycardia.

9. Use of compounds of general formula I according to claim 1 or the physiologically acceptable acid addition salts thereof for the preparation of pharmaceutical compositions for the treatment of tachycardia.

**Claims for the designated contracting state AT**

1. Process for the preparation of substituted 2-phenylamino-imidazolines-(2) of general formula

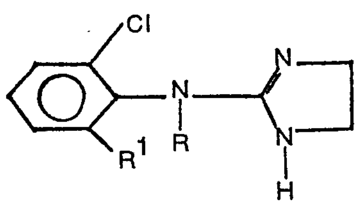

I

wherein $R^1$ represents a chlorine atom or a methyl group and R represents the group $—(CH_2)_2—C(CH_3)=CH_2$, $—(CH_2)_2—CH=CH_2$, $—O—CH_2—CH=CH_2$, $—O—CH_2—C(CH_3)=CH_2$ or $—O—CH_2—CH=CH—CH_3$ and the acid addition salts thereof, characterised in that

a) a 2-phenylimino-imidazolidine of general formula

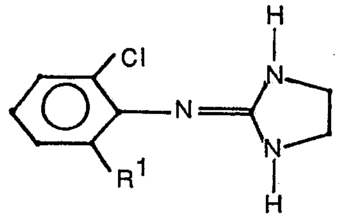

II

wherein $R^1$ is as hereinbefore defined, is reacted with a halide of general formula

Hal—R III

wherein Hal represents a chlorine, bromine or iodine atom and R is as hereinbefore defined, or

b) a compound of general formula

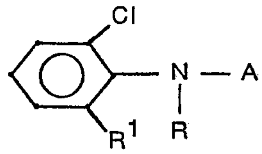

IV

wherein $R^1$ and R are as hereinbefore defined and A represents a cyano group or the group

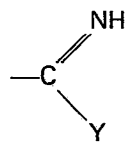

wherein Y represents an alkoxy or alkylthio group with up to 4 carbon atoms or a mercapto or amino group, is reacted with ethylenediamine or the acid addition salts thereof, or

c) to prepare 2-[N-(substituted phenyl)-N-(substituted methoxyamino]-imidazolines-(2) of general formula

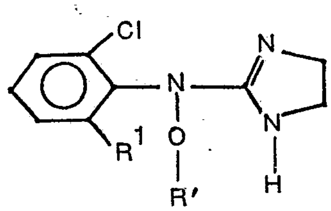

V

wherein $R^1$ is as hereinbefore defined and R′ has the partial formula $—CH_2—CH=CH_2$, $—CH_2—C(CH_3)=CH_2$ or $—CH_2—CH=CH—CH_3$, a 2-[N-(substituted phenyl)-N-hydroxy-amino]-imidazoline-(2) of general formula

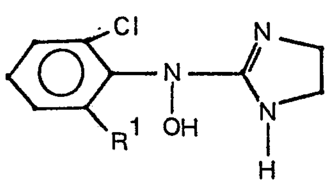

VI

wherein $R^1$ is as hereinbefore defined, is reacted with a halide of general formula

Hal—R′ VII

wherein Hal and R′ are as hereinbefore defined, and if desired the end product obtained by one of these processes is converted into an acid addition salt.

2. Process as claimed in claim 1, characterised in that the reaction is effected at temperatures of from 50 to 150°C or from 60 to 180°C, the latter range applying to variant b) of the process, whilst the former range applies to the two variants a) and c).

3. Process as claimed in claim 1 a) and c), characterised in that the reaction is effected in the presence of a polar or apolar organic solvent.

4. Process as claimed in claim 1 a) and c) to 3, characterised in that the reaction is effected in the presence of an acid-binding agent.

5. Process for the preparation of pharmaceutical compositions, characterised in that one or more compounds of general formula I according to claim 1 or the acid addition salts thereof are combined with conventional galenic excipients, carriers, disintegrants or lubricants or substances for obtained delayed release, to make tablets, capsules, suppositories, solutions or powders.

6. Use of compounds of general formula I according to claim 1 or of the physiologically acceptable acid addition salts thereof for the preparation of pharmaceutical compositions for the treatment of tachycardia.